(19) Europäisches Patentamt
European Patent Office
Office européen des brevets

(11) Publication number: 0 180 356
B1

(12) EUROPEAN PATENT SPECIFICATION

(45) Date of publication of patent specification: 11.01.89

(51) Int. Cl.[4]: C 07 C 43/12, C 07 C 43/225, C 07 C 41/22

(21) Application number: 85307272.6

(22) Date of filing: 10.10.85

# (54) Preparation of alkoxy halides.

(30) Priority: 18.10.84 GB 8426408

(43) Date of publication of application:
07.05.86 Bulletin 86/19

(45) Publication of the grant of the patent:
11.01.89 Bulletin 89/02

(84) Designated Contracting States:
BE CH DE FR GB IT LI NL

(56) References cited:
AU-A- 10 167
CH-A- 385 386
FR-A-2 320 133

Houben-Weyl "Methoden der Organischen Chemie" V.3(1962), pp. 862,863,866

(73) Proprietor: The British Petroleum Company p.l.c.
Britannic House Moor Lane
London EC2Y 9BU (GB)

(72) Inventor: Hodgson, Philip Kenneth Gordon
The British Petroleum Co. p.l.c. Chertsey Road
Sunbury-on-Thames Middlesex, TW16 7LN (GB)
Inventor: Stewart, Nevin John
The British Petroleum Co. p.l.c. Chertsey Road
Sunbury-on-Thames Middlesex, TW16 7LN (GB)

(74) Representative: MacLeod, Malcolm et al
BP INTERNATIONAL LIMITED Patents Division
Chertsey Road
Sunbury-on-Thames Middlesex TW16 7LN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 180 356 B1

Courier Press, Leamington Spa, England.

## Description

This invention relates to a process for the preparation of alkyl alkoxy, aryl alkoxy or alkylaryl alkoxy halides suitable for use as intermediates in the preparation of surfactants.

Numerous attempts have been made to develop surfactant compositions for use in enhanced oil recovery and the patent literature is replete with descriptions of formulations, see for example, USP's 4,424,135, 4,159,037, 4,110,228, 4,066,124 and 4,018,278.

A useful summary of the art is given in Kirk-Othmer's Encyclopedia of Chemical Technology, Third Edition, Volume 17, pages 168—182. This indicates that most compositions contain (a) a main surfactant which is either a petroleum or a synthetic hydrocarbyl sulphonate and (b) co-surfactants which include simple alcohols, ethoxylated alcohols and sulphated ethoxylated alcohols.

It has also been discovered that alkyl and alkylaryl polyalkoxy alkylene sulphonates may be used as co-surfactants. These compounds are generally prepared by a three-stage process. In the first stage of a typical process, an alcohol or alkyl phenol is condensed with an alkylene oxide in the presence of sodium or potassium hydroxide to form an alkoxylate. This is then halogenated by treatment with thionyl or sulphuryl chloride, usually in the absence of a catalyst. Finally the halide is converted to a sulphonate by reaction with sodium sulphite, again, usually in the absence of a catalyst.

GB 1,499,618 discloses that the sulphonation of alkyl or alkyl aryl halides may be carried out under relatively mild conditions in the presence of a quaternary ammonium compound as catalyst.

We have now discovered that the use of such a catalyst in the second stage, the halogenation stage, results in a shorter reaction period and an increased yield of desired product when compared with the uncatalysed reaction.

Thus according to the present invention there is provided a process for the production of an alkyl, aryl or alkylaryl alkoxy halide which process comprises reacting an alkyl, aryl or alkylaryl alkoxy alcohol of formula:

$$R—(OR^1)_mOH,$$

with a halogenating agent in the presence of a quaternary ammonium compound as catalyst; wherein R is an alkyl group containing 1 to 24 carbon atoms, a phenyl group or an alkyl phenyl group of formula:

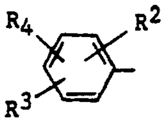

wherein

$R^2$ is an alkyl group containing 8 to 24 carbon atoms and $R^3$ and $R^4$ are hydrogen atoms, or

$R^2$ and $R^3$ are both alkyl groups containing 4 to 12 carbon atoms and $R^4$ is a hydrogen atom, or

$R^2$, $R^3$ and $R^4$ are each alkyl groups wherein the sum of the number of carbon atoms in the groups is in the range 8 to 24;

$R^1$ is an alkylene group containing 2 or 3 carbon atoms; and

m is a number in the range 4 to 15, preferably 4 to 10.

Preferred catalysts are quaternary ammonium compounds of formula:

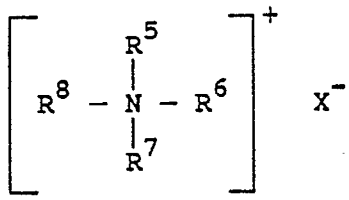

in which $R^5$, $R^6$, $R^7$ and $R^8$ are hydrogen atoms or alkyl, cycloalkyl, aryl or alkylaryl groups containing 1 to 18 carbon atoms, at least one being hydrocarbyl, or two of the radicals $R^5$, $R^6$, $R^7$ and $R^8$, together with the nitrogen atom, represent a heterocyclic ring; and

X denotes an anion, preferably a halide, most preferably a chloride ion.

Preferably $R^5$, $R^6$ and $R^7$ are hydrogen atoms and $R^8$ is an alkyl group containing 1 to 18 carbon atoms.

The catalyst may be bonded onto a polymer such as an ion-exchange resin.

Alternatively, the catalyst may be formed in situ by the reaction of an amine with the halogenating agent. Suitable amines include n-propylamine, n-octadecylamine, ethylenediamine, dimethylamine, trimethylamine and pyridine.

Preferred halogenating agents are chlorinating agents such as thionyl chloride and sulphuryl chloride.

The halogenation reaction is suitably effected at a temperature in the range 30° to 100°C, preferably in the range 75° to 85°C.

Pressure is not a significant parameter and the reaction is therefore most conveniently carried out at ambient pressure.

The molar ratio of alkoxy alcohol to halogenating agent is suitably in the range 1:1 to 1:5, preferably 1:1 to 1:2.5.

The quantity of catalyst employed is preferably in the range 0.1 to 5% by weight, expressed as a percentage by weight of the alkoxy alcohol.

The reaction may be effected in the presence or, preferably, the absence of a solvent. Solvents which may be used include 1,2-dichloroethane, toluene and chloroform.

As a result of the reduced reaction time, the tendency of the polyoxyalkylene chain to cleave noted by Robinson et al, J. Soc. Cosmet. Chem., 31, 329—337, is reduced. Thus both the yield and selectivity as indicated by the matching of the alkoxy distribution in the alcohol and the halide are improved, and product contamination with unwanted by-product is reduced.

The invention is illustrated with reference to the following Examples 1 to 6. Examples 1 and 3 are not in accordance with the invention, however, and are provided for comparison.

Example 1 (For Comparison)

2.25 g (5 mM) of the ethoxyalcohol $C_{12}H_{25}(OCH_2CH_2)_6OH$ was heated under reflux at 86°C with 1.19 g (10 mM) thionyl chloride in 30 ml of anhydrous 1,2-dichloroethane. Thin Layer Chromatography (TLC) indicated that a time in excess of 4 hours was necessary to achieve quantitative chlorination. The product was isolated after 8 hours by pumping in vacuo.

Gas Liquid Chromatography (GLC) indicated that 74.8% 6-ethoxychloride and 22.7% 4-ethoxychloride were produced and that 1,4-dioxan production was consistent with this ethoxy group loss. Mass Spectroscopy confirmed the presence of the ethoxychlorides and 1,4-dioxan. $^1H$ NMR spectroscopy gave a consistent average ethoxylate value for ethoxychloride product of 5.6.

Example 2

1.13 g (2.5 mM) of the ethoxyalcohol $C_{12}H_{25}(OCH_2CH_2)_6OH$ was heated under reflux at 86°C with 0.6 g (5 mM) of thionyl chloride and 10 mg (0.9%) methyltrioctylammonium chloride in 30 ml of anhydrous 1,2-dichloroethane. The product was isolated by evaporation of solvent and volatile reagent.

TLC indicated that quantitative chlorination was complete after 0.5 hours. GLC indicated that 93.5% 6-ethoxychloride and 4.3% 4-ethoxychloride were produced and that 1,4-dioxan production was reduced to 10% of that in Example 1. $^1H$ NMR spectroscopy gave an ethoxychloride ethoxylate value of 5.9.

Example 3 (For Comparison)

61.65 g (0.11 M) of the ethoxyalcohol $C_{18}H_{37}Ph(OCH_2CH_2)_5OH$ was heated with stirring at 80°C wih 14 g (0.12 M) thionyl chloride in the absence of solvent. The product was isolated by pumping in vacuo.

$^{13}C$ NMR spectroscopy indicated 95% conversion to ethoxychloride after 4 hours and GLC indicated the production of 0.08 mole of 1,4-dioxan per mole ethoxyalcohol.

Example 4

87.23 g (0.15 M) of the ethoxyalcohol $C_{18}H_{37}Ph(OCH_2CH_2)_5OH$ was heated with stirring at 80°C with 19.75 g (0.17 M) thionyl chloride and 0.87 g (1%) methyltrioctylammonium chloride in the absence of solvent. The product was isolated by pumping in vacuo.

$^{13}C$ NMR spectroscopy indicated quantitative chlorination after 0.25 hours. GLC indicated the production of 0.01 mole of 1,4-dioxan per mole ethoxyalcohol.

It should be noted when comparing Examples 1 and 2 that addition of the catalyst resulted in at least an eight-fold increase in the chlorination rate in addition to reducing the dioxan production to 1/10 of its previous value.

Examples 3 and 4 show that the addition of the catalyst increased the chlorination rate sixteen-fold and reduced the production of dioxan to 1/8 of its previous value.

Example 5

1.13 g (2.5 mM) of the ethoxyalcohol $C_{12}H_{25}(OCH_2CH_2)_6OH$ was heated under reflux at 86°C with 0.6 g (5 mM) of thionyl chloride and 10 mg (0.9%) of 1-aminooctadecane in 30 ml anhydrous 1,2-dichloroethane. The product was isolated as before.

TLC indicated quantitative chlorination after 1 hour. GLC indicated that 98% 6-ethoxychloride and 2% 4-ethoxychloride were produced.

In comparison with the uncatalysed reaction of Example 1, catalysis by this amine increased the reaction rate at least fourfold and reduced 4-ethoxychloride production by ethoxy chain cleavage to a very low level.

Example 6

1.13 g (2.5 mM) of the ethoxyalcohol $C_{12}H_{25}(OCH_2CH_2)_6OH$ was heated under reflux at 80°C with 0.6 g (5 mM) of thionyl chloride and 10 mg (0.9%) of 1-aminopropane in the absence of a solvent. The product was isolated as before.

TLC indicated complete chlorination after 0.5 hours. GLC indicated that 96% 6-ethoxychloride and 4% 4-ethoxychloride were produced.

In comparison with the uncatalysed reaction of Example 1, the presence of 1-aminopropane increased the rate of reaction at least eightfold and reduced 4-ethxoychloride production by ethoxy chain cleavage from 22.7% to 4%.

**Claims**

1. A process for the production of an alkyl, aryl or alkylaryl alkoxy halide which process comprises reacting an alkyl, aryl, or alkylaryl alkoxy alcohol of formula:

$$R—(OR^1)_m\ OH,$$

wherein R is an alkyl group containing 1 to 24 carbon atoms, a phenyl group or an alkylphenyl group of formula:

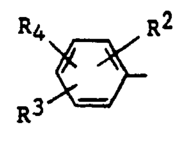

wherein

$R^2$ is an alkyl group containing 8 to 24 carbon atoms and $R^3$ and $R^4$ are hydrogen atoms, or

$R^2$ and $R^3$ are both alkyl groups containing 4 to 12 carbon atoms and $R^4$ is a hydrogen atom, or

$R^2$, $R^3$ and $R^4$ are each alkyl groups wherein the sum of the number of carbon atoms in the groups is in the range 8 to 24;

$R^1$ is an alkylene group containing 2 or 3 carbon atoms; and

m is a number in the range 4 to 15, with a halogenating agent, characterised by the fact that the reaction is carried out in the presence of a quaternary ammonium compound as catalyst.

2. A process according to claim 1 wherein the catalyst is a quaternary ammonium compound of formula:

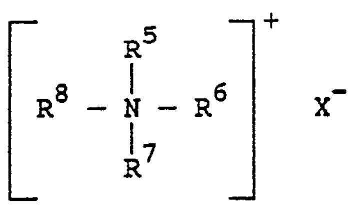

wherein $R^5$, $R^6$, $R^7$ and $R^8$ are hydrogen atoms or alkyl, cycloalkyl, aryl or alkylaryl groups containing 1 to 18 carbon atoms, at least one being hydrocarbyl, or

two of the radicals $R^5$, $R^6$, $R^7$ and $R^8$, together with the nitrogen atom, represent a heterocyclic ring; and

X denotes a halide anion.

3. A process according to claim 2 wherein $R^5$, $R^6$ and $R^7$ are hydrogen atoms and $R^8$ is an alkyl group containing 1 to 18 carbon atoms.

4. A process according to any of the preceding claims wherein the catalyst is formed in situ by the reaction of an amine with the halogenating agent.

5. A process according to any of the preceding claims wherein the halogenating agent is thionyl chloride or sulphuryl chloride.

6. A process according to any of the preceding claims wherein the halogenation reaction is effected at a temperature in the range 30° to 100°C.

7. A process according to any of the preceding claims wherein the molar ratio of alkoxy alcohol to halogenating agent is in the range 1:1 to 1:5.

8. A process according to any of the preceding claims wherein the quantity of catalyst employed is in the range 0.1% to 5% by weight, expressed as a percentage by weight of the alkoxy alcohol.

9. A process according to any of the preceding claims wherein the reaction is carried out in the absence of a solvent.

**Patentansprüche**

1. Verfahren zur Herstellung eines Alkyl-, Aryl- oder Alkylarylalkoxyhalogenids, bei dem ein Alkyl-, Aryl- oder Alkylarylalkoxyalkohol der Formel

$$R{-}(OR^1)_mOH$$

in der

R eine Alkyl-Gruppe mit 1 bis 24 Kohlenstoff-Atomen, eine Phenyl-Gruppe oder eine Alkylphenyl-Gruppe der Formel

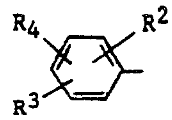

ist, in der

$R^2$ eine Alkyl-Gruppe mit 8 bis 24 Kohlenstoff-Atomen ist und $R^3$ und $R^4$ Wasserstoff-Atome sind oder

$R^2$ und $R^3$ beide Alkyl-Gruppen mit 4 bis 12 Kohlenstoff-Atomen sind und $R^4$ ein Wasserstoff-Atom ist oder

$R^2$, $R^3$ und $R^4$ jeweils Alkyl-Gruppen sind, wobei die Summe der Zahl der Kohlenstoff-Atome in den Gruppen im Bereich von 8 bis 24 liegt,

$R^1$ eine Alkylen-Gruppe mit 2 oder 3 Kohlenstoff-Atomen ist und

m eine Zahl im Bereich von 4 bis 15 ist,

mit einem Halogenierungsmittel umgesetzt wird, dadurch gekennzeichnet, daß die Reaktion in Gegenwart einer quaternären Ammonium-Verbindung als Katalysator durchgeführt wird.

2. Verfahren nach Anspruch 1, worin der Katalysator eine quaternäre Ammonium-Verbindung der Formel

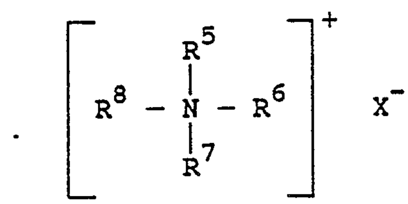

ist, in der

$R^5$, $R^6$, $R^7$ und $R^8$ Wasserstoff-Atome oder Alkyl-, Cycloalkyl-, Aryl- oder Alkylaryl-Gruppen mit 1 bis 18 Kohlenstoff-Atomen sind, wobei wenigstens einer der Reste ein Kohlenwasserstoff-Rest ist, oder

zwei der Reste $R^5$, $R^6$, $R^7$ und $R^8$ zusammen mit dem Stickstoff-Atom einen heterocyclischen Ring bezeichnen, und

X für ein Halogenid-Anion steht.

3. Verfahren nach Anspruch 2, worin $R^5$, $R^6$ und $R^7$ Wasserstoff-Atome sind und $R^8$ eine Alkyl-Gruppe mit 1 bis 18 Kohlenstoff-Atomen ist.

4. Verfahren nach irgendeinem der vorhergehenden Ansprüche, worin der Katalysator in situ durch die Reaktion eines Amins mit dem Halogenierungsmittel gebildet wird.

5. Verfahren nach irgendeinem der vorhergehenden Ansprüche, worin das Halogenierungsmittel Thionylchlorid oder Sulfurylchlorid ist.

6. Verfahren nach irgendeinem der vorhergehenden Ansprüche, worin die Halogenierungs-Reaktion bei einer Temperatur im Bereich von 30°C bis 100°C durchgeführt wird.

7. Verfahren nach irgendeinem der vorhergehenden Ansprüche, worin das Stoffmengen-Verhältnis ("Molverhältnis) des Alkoxyalkohols zu dem Halogenierungsmittel im Bereich von 1:1 bis 1:5 liegt.

8. Verfahren nach irgendeinem der vorhergehenden Ansprüche, worin die eingesetzte Menge des Katalysators im Bereich von 0,1 bis 5 Gew.-%, ausgedrückt · als Prozentsatz des Gewichts des Alkoxyalkohols, liegt.

9. Verfahren nach irgendeinem der vorhergehenden Ansprüche, worin die Reaktion in Abwesenheit eines Lösungsmittels durchgeführt wird.

**Revendications**

1. Procédé pour produire un halogénure d'alcoxy alkyle, d'alcoxy aryle ou d'alcoxy alkylaryle, ce procédé comprenant la réaction d'un alkyl alcoxy alcool, d'un aryl alcoxy alcool ou d'un alkylaryl alcoxy alcool de formule

$$R—(OR^1)_mOH$$

dans laquelle R représente un groupe alkyle contenant 1 à 24 atomes de carbone, un groupe phényle, ou un groupe alkylphényle de formule

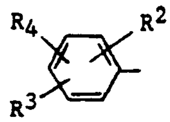

dans laquelle $R^2$ représente un groupe alkyle contenant 8 à 24 atomes de carbone et $R^3$ et $R^4$ représentent chacun un atome d'hydrogène, ou

$R^2$ et $R^3$ représentent chacun un groupe alkyle contenant 4 à 12 atomes de carbone, et $R^4$ est un atome d'hydrogène, ou bien

$R^2$, $R^3$ et $R^4$ représentent chacun un groupe alkyle, la somme du nombre des atomes de carbone dans les groupes se situant entre 8 et 24;

$R^1$ est un groupe alkylène contenant 2 ou 3 atomes de carbone; et

*m* est un nombre valant 4 à 15,

avec un agent d'halogénation,

procédé caractérisé par le fait que l'on conduit la réaction en présence d'un composé d'ammonium quaternaire comme catalyseur.

2. Procédé selon la revendication 1, dans lequel le catalyseur est un composé d'ammonium quaternaire de formule

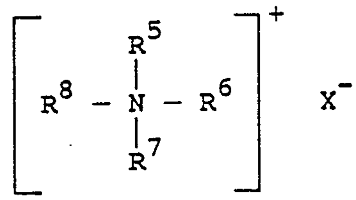

dans laquelle $R^5$, $R^6$, $R^7$ et $R^8$ représentent chacun un atome d'hydrogène ou un groupe alkyle, cycloalkyle, aryle ou alkylaryle contenant 1 à 18 atomes de carbone, au moins l'un de ces symboles représentant un groupe hydrocarbyle, ou bien

deux des radicaux $R^5$, $R^6$, $R^7$ et $R^8$, pris avec l'atome d'azote, représentent un noyau hétérocyclique; et

X désigne un anion halogénure.

3. Procédé selon la revendication 1, dans lequel $R^5$, $R^6$ et $R^7$ représentent chacun un atome d'hydrogène et $R^8$ représente un groupe alkyle contenant 1 à 18 atomes de carbone.

4. Procédé selon l'une quelconque des revendications précédentes, dans lequel le catalyseur est formé sur place par la réaction d'une amine avec l'agent d'halogénation.

5. Procédé selon l'une quelconque des revendications précédentes, dans lequel l'agent d'halogénation est le chloure de thionyle ou le chlorure de sulfuryle.

6. Procédé selon l'une quelconque des revendications précédentes, dans lequel on effectue la réaction d'halogénation en opérant à une température comprise entre 30° et 100°C.

7. Procédé selon l'une quelconque des revendications précédentes, dans lequel le rapport molaire de l'alcoxy alcool à l'agent d'halogénation se situe entre 1:1 et 1:5.

8. Procédé selon l'une quelconque des revendications précédentes, dans lequel la quantité de catalyseur que l'on utilise se situe entre 0,1% et 5% en poids, exprimée en pourcentage pondéral de l'alcoxy alcool.

9. Procédé selon l'une quelconque des revendications précédentes, dans lequel on effectue la réaction en l'absence d'un solvant.